Europäisches Patentamt

European Patent Office

Office européen des brevets

(19)

(11) Veröffentlichungsnummer : **0 047 423**
**B1**

(12) # EUROPÄISCHE PATENTSCHRIFT

(45) Veröffentlichungstag der Patentschrift :
02.05.84

(21) Anmeldenummer : 81106562.2

(22) Anmeldetag : 24.08.81

(51) Int. Cl.³ : **C 07 C109/10, C 08 K 5/25//**
**H01B3/18, H01B3/30**

(54) Verfahren zur Herstellung von N,N'-Bis-salicyloyl-hydrazin und dessen Verwendung.

(30) Priorität : 04.09.80 DE 3033383

(43) Veröffentlichungstag der Anmeldung :
17.03.82 Patentblatt 82/11

(45) Bekanntmachung des Hinweises auf die Patenterteilung : 02.05.84 Patentblatt 84/18

(84) Benannte Vertragsstaaten :
AT BE CH DE FR GB IT LI NL SE

(56) Entgegenhaltungen :
EP-A- 0 025 505
DE-A- 2 327 112
DE-A- 2 703 558
Chemical Abstracts Band 74, Nr. 17, 26. April 1971, Columbus, Ohio, USA S.I. BURMISTROV et al. "Phenolcarboxylic acid hydrazides" Seite 409, Spalte 1, Abstract Nr. 87647r

(73) Patentinhaber : SIEMENS AKTIENGESELLSCHAFT
Berlin und München Wittelsbacherplatz 2
D-8000 München 2 (DE)

(72) Erfinder : v. Gentzkow, Wolfgang, Dr.
Zwetschgenweg 1
D-8524 Kleinsendelbach (DE)

EP 0 047 423 B1

Jouve, 18, rue St-Denis, 75001 Paris, France

# 0 047 423

## Verfahren zur Herstellung von N,N'-Bis-salicyloyl-hydrazin und dessen Verwendung

Die Erfindung betrifft ein Verfahren zur Herstellung von N,N'-Bis-salicyloyl-hydrazin durch katalytische Umsetzung von Salicylsäurealkylester mit Hydrazin oder Salicylsäurehydrazid sowie die Verwendung des derart hergestellten N,N'-Bis-salicyloyl-hydrazins.

Viele organische Materialien, insbesondere Polymere wie Polyolefine, aber auch andere Polymere, wie Polyoxymethylen, Polyamid und ungesättigte Polyesterharze, die heutzutage in der Elektrotechnik für Isolationszwecke verwendet werden, unterliegen in Gegenwart von Kupfer einer beschleunigten thermooxidativen Alterung. Dadurch verschlechtern sich ihre elektrischen und mechanischen Dauergebrauchseigenschaften beträchtlich. Besonders gravierend wird der schädigende Einfluß des Kupfers bei erhöhten Temperaturen, da die Alterungsgeschwindigkeit der Polymeren mit steigenden Temperaturen stark zunimmt.

Insbesondere vernetzte Polyolefine, die in zunehmendem Umfang als Isoliermaterial für Kabel und Leitungen eingesetzt werden, unterliegen in Gegenwart von Kupfer einer stark beschleunigten Alterung und müssen deshalb wirksam gegen den oxidationsbeschleunigenden Einfluß von Kupfer geschützt werden. Dies kann erfahrungsgemäß durch Einbringen einer Folie als Trennschicht zwischen Kupferleiter und Isolierung geschehen oder durch Verwendung verzinnter Leiter; dadurch wird der direkte Kupferkontakt der Isolierung vermieden. Derartige Maßnahmen sind jedoch teuer und fertigungstechnisch sehr aufwendig. So sind beispielsweise beim Einsatz einer Folie als Trennschicht, insbesondere bei kleinen Leiterquerschnitten, nur geringe Fertigungsgeschwindigkeiten möglich.

Ein weiterer, verfahrenstechnisch einfacher Weg zur Erzielung der gewünschten Anforderungen an die Qualität und die Temperaturbelastbarkeit von Polymerwerkstoffen in Kontakt mit Kupfer beinhaltet die Verwendung sogenannter Kupferdesaktivatoren, die die oxidationsbeschleunigende Wirkung von Kupfer auch bei erhöhten Temperaturen inhibieren. Die Stabilisierung von Polymermaterialien durch Kupferdesaktivatoren ist somit eine kostengünstige Maßnahme.

Aus der DE-OS 21 50 131 ist es bekannt, Kupferdesaktivatoren auf der Basis von N,N'-Bis-salicyloyl-hydrazin zu verwenden. Dabei sollen sich insbesondere mehrfach alkyl- oder alkoxysubstituierte Derivate der Basisverbindung für die Stabilisierung von Polyolefinen gegen die schädigende Wirkung von Kupfer und anderen Übergangsmetallen als wirksam erwiesen haben.

Aus der DE-PS 27 03 558 ist es bekannt, daß zur dauerhaften Stabilisierung von mit Kupfer in Berührung stehenden Polymeren eine Kombination aus N,N'-Bis-salicyloyl-hydrazin als Metalldesaktivator und oligomerem 2,2,4-Trimethyl-1,2-dihydrochinolin als Oxidationsinhibitor technisch vorteilhaft ist. Diese Stabilisatorkombination hat sich auch für die Stabilisierung vernetzter Polyolefine als besonders wirksam erwiesen. Die gute Wirksamkeit von N,N'-Bis-salicyloyl-hydrazin als Metalldesaktivator wurde dabei aufgrund umfangreicher Untersuchungen an Modelladern und anhand von Alterungsversuchen an technischen Produkten ermittelt.

Als Metalldesaktivator diente in der genannten Stabilisatorkombination ein technisches Produkt, welches durch Umsetzung von Salicylsäurehydrazid mit Salicylsäure unter Zugabe von Thionylchlorid und Pyridin in Chlorbenzol direkt erhalten wurde und nach Reinigung durch Auswaschen mit Alkohol in einem Reinheitsgrad von 99 % vorlag (vgl. DE-OS 23 27 112).

Die Einarbeitung eines Metalldesaktivators der genannten Art sowie von weiteren Additiven in einen Polymerwerkstoff, wie beispielsweise in ein Polyolefin für Kabel- und Leitungsisolierungen, erfolgt im technischen Maßstab durch übliche Mischverfahren. In der Kabel- und Leitungstechnik werden dabei bevorzugt zunächst sogenannte Konzentrate, bestehend aus Metalldesaktivator bzw. anderen Additiven und Polymermaterial, hergestellt, die dann durch Einmischen in weiteres Polymermaterial zu Isoliermischungen mit den gewünschten Konzentrationen an Metalldesaktivator und den weiteren Additiven verarbeitet werden. Bei der Herstellung der Konzentrate mit dem auf die genannte Weise hergestellten Metalldesaktivator im technischen Maßstab hat sich gezeigt, daß bei den damit befaßten Personen Augenreizungen bzw. Augenschädigungen auftreten.

Um die Ursache der Augenreizungen bzw. Augenschädigungen zu ermitteln, wurden Versuche mit Kaninchen durchgeführt. Da davon ausgegangen werden muß, daß der Kupferdesaktivator bei der technischen Verarbeitung in fester Form, beispielsweise als Staub, in das Auge gelangen kann, wurde bei den Versuchen die feste Substanz in relativ kurzen Zeitabständen, d. h. täglich, mehrfach direkt in das Auge der Versuchstiere eingebracht und dabei wurden die Veränderungen am Auge über einen längeren Zeitraum hinweg beobachtet. Als Ergebnis dieser Versuche wurde festgestellt, daß die Augenreizungen und Augenschädigungen auf das N,N'-Bis-salicyloyl-hydrazin selbst oder auf geringe Mengen an herstellungsbedingten Verunreinigungen zurückzuführen sind.

Der europäischen Patentanmeldung Nr. 80104638.4, veröffentlicht unter der Nr. 25505, kann entnommen werden, daß Augenreizungen bzw. Augenschädigungen nurmehr in stark verminderter Form auftreten, wenn ein N,N'-Bis-salicyloyl-hydrazin zum Einsatz gelangt, das durch Umsetzung von Salicylsäurealkylester mit Hydrazin bzw. Salicylsäure-hydrazid gewonnen wird. Diese Umsetzung kann in Gegenwart nukleophiler und/oder elektrophiler Katalysatoren erfolgen, wobei eine nukleophile Katalyse bevorzugt wird. Als Katalysatoren dienen dabei insbesondere primäre, sekundäre oder tertiäre Amine sowie aminogruppenhaltige Verbindungen, wie beispielsweise stickstoffhaltige Heterocyclen, Amide und

2

Hydrazide, oder die Ammoniumsalze dieser Amine und aminogruppenhaltigen Verbindungen mit anorganischen oder organischen Säuren.

Ein Nachteil der genannten Umsetzung sind — bei katalysatorfreier Arbeitsweise oder bei der Verwendung von Protonensäuren als Katalysatoren — die geringen Ausbeuten. Bei der Verwendung nukleophiler Katalysatoren der vorstehend genannten Art fällt das Rohprodukt andererseits oft in verunreinigter, durch Zusatzprodukte gelblich gefärbter Form an und muß nach üblichen Reinigungsmethoden, wie Umkristallisieren, weiter aufgearbeitet werden.

Aufgabe der Erfindung ist es, das Verfahren der eingangs genannten Art in der Weise auszugestalten, daß nicht nur ein N,N'-Bis-salicyloyl-hydrazin mit verminderter Augenreizung erhalten wird, sondern das Produkt auch in hoher Ausbeute und Reinheit anfällt.

Dies wird erfindungsgemäß dadurch erreicht, daß Hydrazin bzw. Salicylsäurehydrazid in einem 1- bis 10fachen Überschuß an Salicylsäurealkylester in Gegenwart eines Halogenides, Hydroxides oder Oxides von Bor, Aluminium oder Zink auf Temperaturen bis zu 150 °C erhitzt wird.

Überraschenderweise hat sich nämlich gezeigt, daß bei der Umsetzung von Hydrazin bzw. Salicylsäurehydrazid mit Salicylsäurealkylester dann ein N,N'-Bis-salicyloyl-hydrazin in hohen Ausbeuten und hoher Reinheit anfällt, wenn ohne Lösungsmittel in einem Überschuß von flüssigem Salicylsäurealkylester bei Temperaturen unterhalb 150 °C gearbeitet und ein elektrophiler Katalysator der vorstehend genannten Art eingesetzt wird. Die für eine rasche Umsetzung benötigten Katalysatorkonzentrationen liegen dabei — je nach Wirksamkeit — vorzugsweise zwischen 0,01 und 5 Masse-%, bezogen auf die Gesamtmasse der Reaktionsmischung.

Bei der Verwendung von Aluminiumoxid oder Aluminiumoxidhydraten ist die Katalysatorkonzentration nur hinsichtlich der für die heterogene Katalyse erforderlichen Katalysatoroberfläche von Bedeutung. Ist eine große spezifische Katalysatoroberfläche vorhanden, dann können bereits sehr geringe Katalysatormengen hohe Ausbeuten bewirken. Wichtig ist allerdings eine hohe Reinheit des eingesetzten Aluminiumoxids bzw. Aluminiumoxidhydrats, da bereits geringe Mengen an Schwermetallverunreinigungen zu einem gefärbten N,N'-Bis-salicyloyl-hydrazin führen. Um eine einfache Trennung des während der Umsetzung ausfallenden Produktes vom festen Katalysator zu ermöglichen, hat es sich ferner bewährt, das Aluminiumoxid durch Oxidation als Überzug auf Aluminiumteilen im Reaktor (Innenwand oder Rührer) zu erzeugen oder den Katalysator in Form eloxierter Aluminiumfolien im Reaktor zu befestigen.

Beim erfindungsgemäßen Verfahren hat es sich als zweckmäßig erwiesen, Salicylsäurealkylester einzusetzen, welche bei Raumtemperatur flüssig sind. Vorzugsweise werden bei diesem Verfahren deshalb Salicylsäureester verwendet, deren (alkoholischer) Alkylrest 1 bis 5 Kohlenstoffatome aufweist.

Ein besonders reines, farbloses N,N'-Bis-salicyloyl-hydrazin in praktisch theoretischer Ausbeute wird erhalten, wenn — ausgehend von Salicylsäuremethylester — in einem Überschuß desselben (300 bis 350 % der stöchiometrisch erforderlichen Menge) bei Temperaturen von maximal 145 °C gearbeitet und Borsäure als Katalysator in einer Konzentration von ca. 0,5 bis 1 %, bezogen auf die Gesamtmasse der Reaktionsmischung, eingesetzt wird. Die Verwendung von Borsäure, d. h. $H_3BO_3$ bzw. $B(OH)_3$, hat sich als vorteilhaft erwiesen, da sie sich während der Umsetzung in Form von Borsäuretrimethylester verflüchtigt und auf diese Weise ein besonders reines, von Katalysatorresten freies Produkt erhalten wird.

Eine besonders vorteilhafte Variante der Katalyse mit Borsäure ist der Einsatz von Bortrioxid $B_2O_3$. Bortrioxid setzt sich nämlich während des Reaktionsablaufs mit geringen Mengen Wasser zu Borsäure um. Damit werden ein gut kontrollierbarer Ablauf der Gesamtreaktion und besonders hohe Ausbeuten sichergestellt. Wird bei der Herstellung von N,N'-Bis-salicyloyl-hydrazin von Salicylsäuremethylester und Hydrazinhydrat ausgegangen und mit $B_2O_3$ bzw. $B(OH)_3$ oder $Al_2O_3$ katalysiert, so ist die Synthese apparativ sehr einfach (« Eintopfreaktion ») und großtechnisch leicht durchführbar und — bei Redestillation der unumgesetzten Reaktanten — äußerst kostengünstig.

Darüber hinaus besitzt das nach dem erfindungsgemäßen Verfahren hergestellte N,N'-Bis-salicyloyl-hydrazin die bisher geringste augenreizende bzw. augenschädigende Wirkung, was auf die hohe Reinheit und die günstige Kristallform (abgerundete Ecken und Kanten) des während der Umsetzung ausfallenden Produktes zurückzuführen sein dürfte.

Das erfindungsgemäß synthetisierte Produkt wird besonders vorteilhaft im Gemisch mit üblichen phenolischen oder aminischen Oxidationsinhibitoren als Kupferdesaktivator eingesetzt ; insbesondere dient dieses Produkt zur Stabilisierung von Kunststoffen und Kunststoffvorprodukten gegen oxidativen Abbau. Eine besonders synergistische Wirkung wird im Gemisch mit oligomerem 2,2,4-Trimethyl-1,2-dihydrochinolin erzielt. Die Verarbeitung des erfindungsgemäß synthetisierten Metalldesaktivators zur Stabilisierung von Kunststoffen kann nach der üblichen Mischtechnik, beispielsweise über Konzentrate, insbesondere aber auch durch Direktdosierung eines phlegmatisierten, d. h. durch geeignete flüssige Zusätze verarbeitungsfreundlicher eingestellten Metalldesaktivators, erfolgen.

Mit dem erfindungsgemäß synthetisierten Kupferdesaktivator können vernetzte und unvernetzte Thermoplaste, wie vernetzte und unvernetzte Polyolefine, und Elastomere sowie Reaktionsharze in gehärtetem und ungehärtetem Zustand vorteilhaft gegen schädliche katalytische Einflüsse von Kupfer geschützt werden. Polymere, die den erfindungsgemäßen Metalldesaktivator enthalten, können besonders vorteilhaft als Kabel- und Leitungsisolierungen in der Starkstromtechnik und Nachrichtentechnik eingesetzt werden. Darüber hinaus kann der erfindungsgemäß synthetisierte Kupferdesaktivator auch zur

3

**0 047 423**

Stabilisierung von Polymerwerkstoffen, von Isolierölen und von Schmierfetten, die mit Kupfer in Kontakt stehen oder Kupferionen enthalten, Verwendung finden. Derartige Isolieröle und Schmierfette gelangen insbesondere in der Elektrotechnik zum Einsatz.

Anhand von Beispielen soll die Erfindung noch näher erläutert werden.

### Beispiel 1

152 Gew.-Tle. Salicylsäurehydrazid werden mit 380 Gew.-Tln. Salicylsäuremethylester und einer entsprechenden Menge Katalysator (siehe Tabelle 1) unter Rühren auf eine Temperatur von ca. 140-145 °C erhitzt. Während der Umsetzung entstehendes Methanol wird über eine Kolonne abdestilliert. Nach ca. 1 h beginnt die Reaktionsmischung durch das ausfallende N,N′-Bis-salicyloyl-hydrazin dickflüssig zu werden. Nach 2 h ist die Reaktion beendet. Die nun sehr dickflüssige Reaktionsmischung wird auf 70 °C abgekühlt, mit dem gleichen Volumen Alkohol (Methanol oder Äthanol) versetzt und ca. 30 min bei 60-70 °C gerührt. Nach dem Abkühlen wird abgesaugt, mit Alkohol gewaschen und im Vakuum getrocknet. Es wird ein rein weißes Produkt mit einem Zersetzungsbereich von 305-310 °C erhalten. Sie Ausbeuten sind in Tabelle 1 angegeben.

### Beispiel 2

50 Gew.-Tle. Hydrazinhydrat werden mit 532 Gew.-Tln Salicylsäuremethylester 2 h unter Rückfluß gekocht, wobei gerührt wird. Nach ca. 1 h beginnt Salicylsäurehydrazid auszufallen. Nach 2 h wird Wasser und gebildetes Methanol über eine Kolonne abdestilliert und die Reaktionsmischung mit einer entsprechenden Menge Katalysator (siehe Tabelle 1) versetzt und unter Rühren auf eine Temperatur von ca. 140-145 °C erhitzt. Dabei löst sich ein Großteil des vorher ausgefallenen Salicylsäurehydrazids wieder auf. Während des weiteren Verlaufs der Umsetzung gebildetes Methanol wird kontinuierlich über eine Kolonne abdestilliert. Nach ca. 1 h beginnt die Reaktionsmischung durch ausfallendes N,N′-Bis-salicyloyl-hydrazin dickflüssig zu werden. Nach ca. 2 h ist die Reaktion beendet. Die nun sehr dickflüssige Reaktionsmischung wird auf 70 °C abgekühlt, mit dem gleichen Volumen Alkohol (Methanol oder Äthanol) versetzt und ca. 30 min bei 60-70 °C gerührt. Nach dem Abkühlen wird abgesaugt, mit Alkohol gewaschen und im Vakuum getrocknet. Es wird ein rein weißes Produkt mit einem Zersetzungsbereich von 305-310 °C erhalten. Die Ausbeuten sind in Tabelle 1 angegeben.

### Tabelle 1

| Katalysator | Menge (Gew.-Tle.) | Verfahren n. Beispiel | Ausbeute (%) |
|---|---|---|---|
| $BCl_3$ | 12 (*) | 2 | 66 (**) |
| $BF_3 \cdot (C_2H_5)_2O$ | 14 | 2 | 62 (**) |
| $B(OH)_3$ | 6 | 1 oder 2 | 93 bzw. 88 |
| $B_2O_3$ | 3 | 1 oder 2 | 94 bzw. 93 |
| $Al_2O_3$ (gekörnt) | 10 | 1 oder 2 | 84 (**) bzw. 78 (**) |
| $Al_2O_3 \cdot 3 H_2O$ (gekörnt) | 15 | 1 oder 2 | 82 (**) bzw. 74 (**) |
| $ZnCl_2$ | 14 | 2 | 60 |

(*) Berechneter Wert aus der gasförmig eingeleiteten Menge.
(**) Die Ausbeuten können bei Verlängerung der Reaktionsdauer erhöht werden.

### Beispiel 3

152 Gew.-Tle. Salicylsäurehydrazid werden mit 380 Gew.-Tln. Salicylsäuremethylester in einem Gefäß mit einem großflächigen eloxierten Aluminiumrührer unter Rühren auf eine Temperatur von ca. 140-145 °C erhitzt. Nach ca. 2 h beginnt die Reaktionsmischung durch ausfallendes N,N′-Bis-salicyloyl-hydrazin dickflüssig zu werden. Nach 3 h wird die dickflüssige Reaktionsmischung auf 70 °C abgekühlt, mit dem gleichen Volumen Alkohol (Methanol oder Äthanol) versetzt und ca. 30 min bei 60-70 °C gerührt. Nach dem Abkühlen wird abgesaugt, mit Alkohol gewaschen und im Vakuum getrocknet. Es wird mit einer Ausbeute von ca. 80 %, bezogen auf Salicylsäurehydrazid, ein rein weißes Produkt mit einem Zersetzungsbereich von 305-310 °C erhalten. Die Ausbeute kann bei Vergrößerung der Katalysatoroberfläche oder bei Verlängerung der Reaktionszeit noch erhöht werden.

### Beispiel 4

Wird die Umsetzung nach Beispiel 2 entsprechend Beispiel 3, d. h. unter Verwendung eines großflächigen eloxierten Aluminiumrührers, durchgeführt, so wird — in einer Ausbeute von 76 % — ein rein weißes Produkt mit einem Zersetzungsbereich von 305-310 °C erhalten. Die Ausbeute kann bei Vergrößerung der Katalysatoroberfläche oder bei Verlängerung der Reaktionszeit noch erhöht werden.

4

Die Überprüfung der augenreizenden bzw. augenschädigenden Wirkung des nach dem erfindungsgemäßen Verfahren hergestellten N,N'-Bis-salicyloyl-hydrazins erfolgte nach der bereits erwähnten Testmethode. Dabei wurde je Versuchsreihe 6 Kaninchen 5 Tage lang täglich 0,1 g Substanz in den Bindehautsack des rechten Auges appliziert. Bei je 3 der 6 Versuchstiere einer Versuchsreihe wurde kurze Zeit nach der Applikation der Bindehautsack mit physiologischer Kochsalzlösung gespült, wobei auf völlige Entfernung der Substanz geachtet wurde. Die restlichen 3 Versuchstiere der gleichen Versuchsreihe wurden nicht weiter behandelt.

Bei den Kaninchen, deren Augen nach der Applikation von N,N'-Bis-salicyloyl-hydrazin nicht gespült wurden, traten mit dem gemäß vorliegender Erfindung hergestellten Produkt nur eine vorübergehende, relativ schwache Rötung der Bindehaut und eine geringe hauchige Trübung der Hornhaut auf. Bei den Kaninchen, deren Augen nach der Behandlung gespült wurden, konnte neben einer vorübergehenden schwachen Reizung während des Applikationszeitraumes keine Schädigung der Augen nachgewiesen werden.

## Ansprüche

1. Verfahren zur Herstellung von N,N'-Bis-salicyloyl-hydrazin durch katalytische Umsetzung von Salicylsäurealkylester mit Hydrazin oder Salicylsäurehydrazid, dadurch gekennzeichnet, daß Hydrazin bzw. Salicylsäurehydrazid in einem 1- bis 10-fachen Überschuß an Salicylsäurealkylester in Gegenwart eines Halogenides, Hydroxides oder Oxides von Bor, Aluminium oder Zink auf Temperaturen bis zu 150 °C erhitzt wird.

2. Verfahren nach Anspruch 1, dadurch gekennzeichnet, daß der Katalysator in einer Menge von 0,01 bis 5 Gew.-% eingesetzt wird, bezogen auf das Gesamtgewicht.

3. Verfahren nach Anspruch 1 oder 2, dadurch gekennzeichnet, daß ein 2- bis 3facher Überschuß an Salicylsäurealkylester verwendet wird.

4. Verfahren nach einem der Ansprüche 1 bis 3, dadurch gekennzeichnet, daß als Katalysator Borsäure $B(OH)_3$ oder Bortrioxid $B_2O_3$ verwendet wird.

5. Verwendung des nach dem Verfahren nach einem oder mehreren der Ansprüche 1 bis 4 hergestellten N,N'-Bis-salicyloyl-hydrazins als Metalldesaktivator für mit Kupfer in Berührung stehende oder Kupfer bzw. Kupferionen enthaltende Kunststoffe, Kunststoffvorprodukte, Isolieröle und Schmierfette.

## Claims

1. A process for the production of N,N'-bis-salicyloyl hydrazine by the catalytic reaction of a salicylic acid alkyl ester with hydrazine or salicylic acid hydrazide, characterised in that hydrazine or salicylic acid hydrazide is heated to a temperature of up to 150 °C with salicylic acid alkyl ester in an excess of from 1 to 10 times, in the presence of a halide, hydroxide or oxide of boron, aluminium or zinc.

2. A process according to Claim 1, characterised in that the catalyst is introduced in an amount of 0.01 to 5 % of the total weight.

3. A process according to Claim 1 or Claim 2, characterised in that a 2- to 3-fold excess of salicylic acid alkyl ester is used.

4. A process according to one of Claims 1 to 3, characterised in that boric acid $B(OH)_3$, or boron trioxide $B_2O_3$ is used as a catalyst.

5. The use of N,N'-bis-salicyloyl hydrazine produced in accordance with the process as claimed in one or more of Claims 1 to 4 as a metal deactivator for plastics, plastics preproducts, insulating oils and lubricating greases which are in contact with copper, or contain copper or copper ions.

## Revendications

1. Procédé de préparation de N,N'-bis-salicyloyl-hydrazine par réaction catalytique d'un ester alcoylique d'acide salicylique sur l'hydrazine, ou sur l'hydrazide d'acide salicylique, caractérisé en ce qu'il consiste à chauffer à des températures allant jusqu'à 150 °C, l'hydrazine ou l'hydrazide d'acide salicylique en un excès de 1 à 10 fois par rapport à l'ester alcoylique d'acide salicylique, en présence d'un halogénure, d'un hydroxyde ou d'un oxyde de bore, d'aluminium ou de zinc.

2. Procédé suivant la revendication 1, caractérisé en ce qu'il consiste à engager le catalyseur en une quantité représentant de 0,01 à 5 % du poids total.

3. Procédé suivant la revendication 1 ou 2, caractérisé en ce qu'il consiste à utiliser un excès de 2 à 3 fois d'ester alcoylique d'acide salicylique.

4. Procédé suivant l'une des revendications 1 à 3, caractérisé en ce qu'il consiste à utiliser comme catalyseur de l'acide borique $B(OH)_3$ ou du trioxyde de bore $B_2O_3$.

5. Utilisation de la N,N'-bis-salicyloyl-hydrazine préparée par le procédé suivant l'une ou plusieurs des revendications 1 à 4, comme désactivateur de métal pour des matières plastiques, des précurseurs de matières plastiques, des huiles isolantes, et des graisses lubrifiantes, en contact avec du cuivre, ou contenant du cuivre ou des ions cuivre.